# FASCICULE DE BREVET EUROPEEN

(11) **EP 1 741 785 B1**
(45) Date de publication et mention de la délivrance du brevet: **01.10.2014**
(21) Numéro de dépôt: 05014998.8
(22) Date de dépôt: 01.10.1998
(51) Int. Cl.: C12N 15/34, C07K 14/01, A61K 39/12, A61K 48/00, G01N 33/53

(54) **Circovirus porcins, acides nucléiques, polypeptides et vaccins**
Schweinecircoviren, Nukleinsäuren, Polypeptide und Impfstoffe
Porcine circoviruses, nucleic acids, polypeptides and vaccines

(30) Priorité: 03.10.1997 FR 9712382; 22.01.1998 FR 9800873; 20.03.1998 FR 9803707
(43) Date de publication de la demande: 10.01.2007
(62) Demande divisionnaire de: 02017134.4
(73) Titulaire: MERIAL, 69007 Lyon (FR); The Queen's University of Belfast, Belfast BT4 3SD (GB); The University of Saskatchewan, Saskatoon, Saskatchewan S7W 5B4 (CA)
(72) Inventeur: Allan, Gordon, Belfast BT5 7AQ (GB); Meehan, Brian, Belfast BT1 3LX (GB); Clark, Edward, Saskatoon Saskatchewan S7J 214 (CA); Ellis, John, Saskatoon Saskatchewan S7N 0M3 (CA); Haines, Déborah, Saskatoon Saskatchewan S7N 9M3 (CA); Hassard, Lori, Saskatoon Saskatchewan S7K 2A0 (CA); Harding, John, Humboldt Saskatchewan 2 S0K 2A0 (CA); Charreyre, Catherine Elisabeth, 69720 Saint-Laurent de Mure (FR); Chappuis, Gilles Emile, 69600 Oullins (FR); McNeilly, Francis, Newtownards BT3 4NH (GB)
(74) Mandataire: Cabinet Plasseraud

(56) Documents cités:
- WO-A2-99/29717
- WO-A2-99/29871
- MEEHAN B.M. ET AL.: "Sequence of porcine circovirus DNA: affinities with plant circoviruses" JOURNAL OF GENERAL VIROLOGY, vol. 78, no. 1, January 1997 (1997-01), pages 221-227, XP002068398
- MANKERTZ A. ET AL.: "Mapping and characterization of the origin of DNA replication of porcine circovirus" JOURNAL OF VIROLOGY, vol. 71, no. 3, March 1997 (1997-03), pages 2562-2566, XP002078782
- NAYAR G.P.S. ET AL.: "Detection and characterization of porcine circovirus associated with postweaning multisystemic wasting syndrome in pigs" CANADIAN VETERINARY JOURNAL - REVUE VETERINAIRE CANADIENNE, vol. 38, June 1997 (1997-06), pages 385-386, XP002068396
- MEEHAN B.M. ET AL.: "Characterization of novel circovirus DNAs associated with wasting syndromes in pigs" JOURNAL OF GENERAL VIROLOGY, vol. 79, no. 9, September 1998 (1998-09), pages 2171-2179, XP002090386
- HAMEL A.L. ET AL.: "Nucleotide sequence of Porcine Circovirus associated with postweaning multisystemic wasting syndrome in pigs" JOURNAL OF VIROLOGY, vol. 72, no. 6, June 1998 (1998-06), pages 5262-5267, XP002078783
- MOROZOV I. ET AL.: "Detection of a novel strain of porcine circovirus in pigs with postweaning multisystemic wasting syndrome" JOURNAL OF CLINICAL MICROBIOLOGY, vol. 36, no. 9, September 1998 (1998-09), pages 2535-2541, XP002090921
- ALLAN G M ET AL: "PORCINE CIRCOVIRUSES: A REVIEW" JOURNAL OF VETERINARY DIAGNOSTIC INVESTIGATION, AAVLD, COLUMBIA, MO, US, vol. 12, no. 1, January 2000 (2000-01), pages 3-14, XP001023034 ISSN: 1040-6387
- HARDING: PROCEEDINGS, AMERICAN ASSOCIATION OF SWINE PRACTIONERS, 1997, XP000000503,
- CLARK: PROCEEDINGS, WESTERN CANADIAN ASSOCIATION OF SWINE PRACTITIONERS, 1997,

## Description

La présente invention est relative à de nouvelles souches de circovirus porcin (PCV pour *Porcine CircoVirus*) responsables du syndrome PMWS *(Porcine Multisystemic Wasting Syndrome* ou *Post-Weaning Multisystemic Wasting Syndrome* encore appelé syndrome de dépérissement généralisé de post-sevrage), à des réactifs et méthodes permettant leur détection, à des méthodes de vaccination et à des vaccins, ainsi qu'à des méthodes de production de ces réactifs et vaccins.

Le PCV a été à l'origine détecté comme contaminant non cytopathogène dans des lignées cellulaires de reins de porcs PK/15. Ce virus a été classé parmi les Circoviridae avec le virus de l'anémie du poulet (CAV pour *Chicken Anemia Virus)* et le virus PBFDV (*Pscittacine Beak and Feather Disease Virus).* Il s'agit de petits virus (de 15 à 24 nm) non enveloppés dont la caractéristique commune est de contenir un génome sous forme d'un ADN simple brin circulaire de 1,76 à 2,31 kb. On a d'abord pensé que ce génome codait pour un polypeptide d'environ 30 kDa (Todd et al., Arch Virol 1991, 117: 129-135). Des travaux récents ont toutefois montré une transcription plus complexe (Meehan B. M. et al., 1997, 78: 221-227). Par ailleurs, on ne connaît pas d'homologies significatives de séquence nucléotidique ni de déterminants antigéniques communs entre les trois types de circovirus connus.

Le PCV issu des cellules PK/15 est considéré comme n'étant pas pathogène. On en connaît la séquence d'après B. M. Meehan et al., J Gen Virol 1997 (78) 221-227. Ce n'est que très récemment que des auteurs ont pensé que des souches de PCV pourraient être pathogènes et associées au syndrome PMWS (Gupi P. S. Nayar et al., Can Vet J , vol. 38, 1997: 385-387 et Clark E. G., Proc Am Assoc Swine Prac 1997: 499-501). Nayar et al. ont détecté de l'ADN de PCV chez des porcs présentant le syndrome PMWS par des techniques de PCR. Aucune souche sauvage de PCV n'a toutefois été isolée et purifiée à ce jour.

Le syndrome PMWS détecté au Canada, aux Etats-Unis et en France se caractérise au plan clinique par une perte progressive de poids et par des manifestations telles que tachypnée, dyspnée et jaunisse. Au plan pathologique, il se traduit par des infiltrations lymphocytaires ou granulomateuses, des lymphadénopathies et, plus rarement, par des hépatites et néphrites lymphocytaires ou granulomateuses (Clark E. G., Proc. Am. Assoc. Swine Prac. 1997: 499-601 ; La Semaine Vétérinaire n° 26, supplément à La Semaine Vétérinaire 1996 (834) ; La Semaine Vétérinaire 1997 (857): 54 ; Gupi P. S. Nayar et al., Can Vet J , vol. 38, 1997: 385-387).

La déposante a réussi à isoler cinq souches nouvelles de PCV à partir de prélèvements pulmonaires ou ganglionnaires provenant d'élevages situés au Canada, aux Etats-Unis (Californie) et en France (Bretagne), ci-après dénommés circovirus selon l'invention. Ces virus ont été mis en évidence dans des lésions de porcs atteints du syndrome PMWS, mais pas chez des porcs sains.

La déposante a en outre séquencé le génome de quatre de ces souches, à savoir les souches provenant du Canada et des Etats-Unis ainsi que deux souches française. Les souches présentent entre elles une très forte homologie au niveau nucléotidique dépassant 96 % et beaucoup plus faible avec la souche PK/15, environ 76 %. Les nouvelles souches peuvent donc être considérées comme représentatives d'un nouveau type de circovirus porcin, dénommé ici type II, le type I étant représenté par la PK/15.

La présente invention est définie par les revendications le circovirus porcin de groupe II, tel que défini ci-dessus, isolé ou sous forme de préparation purifiée est decrit.

Il est également décrit tout circovirus porcin susceptible d'être isolé d'un échantillon physiologique ou d'un prélèvement tissulaire, notamment de lésions, d'un porc malade présentant le syndrôme PMWS, notamment en suivant la méthode décrite dans les exemples, en particulier circovirus du type II.

La présente invention décrit plus particulièrement des préparations purifiées de cinq souches, qui ont été déposées auprès de l'ECACC (European Collection of Cell Cultures, Centre for Applied Microbiology & Research, Porton Down, Salisbury, Wiltshire SP4 OJG, Royaume-Uni) le jeudi 2 octobre 1997:
- n° d'accès V97100219 (appelé ici Imp.1008PCV)
- n° d'accès V97100218 (appelé ici Imp.1010PCV)
- n° d'accès V97100217 (appelé ici Imp.999PCV).
   et, le vendredi 16 janvier 1998 :
- n° d'accès V98 011608 (appelé ici Imp 1011-48285)
- n° d'accès V98 011609 (appelé ici Imp 1011-48121)

L'invention entend considérer les circovirus porcins isolés d'un porc malade et/ou les circovirus ayant une parenté sérologique significative avec les souches de l'invention et/ou les circovirus ayant une hybridation croisée avec les souches de l'invention dans des conditions de stringence telles qu'il n'y a pas d'hybridation avec la souche PCV PK/15.

Les souches virales isolées d'un échantillon physiologique ou d'un prélèvement tissulaire, notamment d'une lésion, d'un porc présentant le syndrome PMWS peuvent être avantageusement propagées sur des lignées cellulaires telles que notamment des lignées cellulaires de rein de porc, en particulier cellules PK/15 indemnes de contamination (en particulier pour PCV, ainsi que pour pestivirus, adénovirus porcin et parvovirus porcin) en vue de leur multiplication ou spécifiquement pour la production d'antigène, entier (e.g. virus) et/ou sous-unités (e.g. polypeptides).

De manière très remarquable et inattendue, ces isolats se sont révélés très productifs en culture sur cellules PK/15, ce qui présente des avantages indéniables pour la production de virus ou d'antigène, en particulier pour la production de vaccin inactivé.

Sont aussi décrites des préparations de circovirus isolés après passages sur cellules, notamment lignées cellulaires, e.g. cellules PK, cultivées in vitro en étant infectées par l'un au moins des circovirus décrits ici ou de tout circovirus porcin susceptible d'être isolé d'un échantillon physiologique ou d'un prélèvement tissulaire, notamment de lésions, d'un porc présentant le syndrôme PMWS. Sont aussi décrits les surnageants ou extraits de culture, éventuellement purifiés par des techniques standards, et de manière générale toute préparation antigénique obtenue à partir des cultures in vitro.

Sont aussi décrits les principes actifs immunogènes et les vaccins contenant au moins un antigène tel que défini supra.

Il peut s'agir de principes actifs immunogènes à base de virus entiers vivants atténués, ou vaccins préparés avec ces principes actifs, l'atténuation étant effectuée selon les méthodes usuelles, e.g. par passage sur cellules, de préférence par passage sur des cellules de porc, notamment des lignées, telles que les cellules PK/15 (par exemple de 50 à 150, notamment de l'ordre de 100, passages). Ces vaccins comprennent en général un véhicule ou diluant acceptable sur le plan vétérinaire, éventuellement un adjuvant acceptable sur le plan vétérinaire ainsi qu'éventuellement un stabilisateur de lyophilisation.

Ces préparations antigéniques et vaccins comprendront de préférence de 10³ à 10⁶TCID50.

Il peut aussi s'agir de principes actifs immunogènes ou de vaccins à base d'antigène de circovirus selon l'invention, à l'état inactivé. Les vaccins comprennent en outre un véhicule ou diluant acceptable sur le plan vétérinaire, avec éventuellement en plus un adjuvant acceptable sur le plan vétérinaire.

Les circovirus, avec les fractions qui peuvent être présentes, sont inactivés selon les techniques connues de l'homme du métier. L'inactivation sera effectuée de préférence par voie chimique, e.g. par exposition de l'antigène à un agent chimique tel que le formaldéhyde (formol), le paraformaldéhyde, la β-propiolactone ou l'éthylène imine ou ses dérivés. La méthode d'inactivation préférée sera ici l'exposition à un agent chimique et en particulier à l'éthylène imine ou à la ß-propiolactone.

De préférence, les vaccins inactivés divulgués seront adjuvés, avantageusement en étant présentés sous forme d'émulsions, par exemple eau-dans-l'huile ou huile-dans-l'eau, selon les techniques bien connues de l'homme du métier. Le caractère adjuvant pourra aussi provenir de l'incorporation au principe actif d'un composé adjuvant usuel.

Parmi les adjuvants qui peuvent être utilisés, on peut citer à titre d'exemple l'hydroxyde d'alumine, les saponines (e.g. Quillaja saponin ou Quil A ; voir Vaccine Design, The Subunit and Adjuvant Approach, 1995, édité par Michael F. Powel et Mark J. Newman, Plennum Press, New-York and London, p 210), l'Avridine® (Vaccine Design p 148), le DDA (Diméthyldioctadécylammonium bromide, Vaccine Design p 167), le Polyphosphazene (Vaccine Design p 204), ou encore des émulsions huile-dans-l'eau à base d'huile minérale, de squalane (e.g. émulsion SPT, Vaccine Design p 147), de squalène (e.g. MF59, Vaccine Design p 183), ou eau-dans-l'huile à base d'huile métabolisable (de préférence selon WO-A-94 20071) ainsi que les émulsions décrites dans US-A-5 422 109. On peut aussi choisir des associations d'adjuvants, par exemple Avridine® ou DDA associé à une émulsion.

Ces vaccins comprendront de préférence de 10⁶ à 10⁸ TCID50.

Les adjuvants du vaccin vivant pourront être choisis parmi ceux donnés pour le vaccin inactivé. On préférera les émulsions. A celles indiquées pour le vaccin inactivé, on peut rajouter celles décrites dans WO-A-9416681.

Comme stabilisateur de lyophilisation, on peut citer à titre d'exemple le SPGA (Bovarnik et al., J. Bacteriology 59, 509, 950), des hydrates de carbone tels que le sorbitol, mannitol, amidon, saccharose, dextran ou glucose, des protéines telles que l'albumine ou la caséine, des dérivés de ces composés, ou des tampons tels que des tampons de phosphates de métaux alcalins.

La déposante a en outre obtenu le génome de quatre des isolats, identifiés SEQ ID NO: 1 à 4 et éventuellement 6. fragment d'ADN contenant

Il est également décrit un fragment d'ADN contenant tout ou partie de l'une de ces séquences. Sont de soi automatiquement recouvertes les séquences équivalentes, c'est-à-dire les séquences ne changeant pas la fonctionnalité ni la spécificité de souche de la séquence décrite ni des polypeptides codés par cette séquence. Seront bien entendu incluses les séquences différant par dégénérescence du code.

Sont également recouvertes les séquences équivalentes en ce sens qu'elles sont capables de s'hybrider à la séquence supra dans des conditions de stringence élevées et/ou ont une forte homologie avec les souches de l'invention et appartiennent au groupe Il défini plus haut.

Ces séquences et leurs fragments sont utilisés pour l'expression in vitro ou in vivo de polypeptides à l'aide de vecteurs appropriés.

En particulier, des cadres ouverts de lecture, formant des fragments d'ADN selon l'invention, utilisables à cet effet ont été identifiés sur la séquence génomique des circovirus de type II. Est divulgué tout polypeptide contenant au moins un de ces cadres ouverts de lecture (séquence en acides aminés correspondante). L'invention concerne une protéine formée essentiellement par les COL4, COL7, COL10 ou COL13.

Pour l'expression de sous-unités in vitro, comme moyen d'expression on aura de préférence recours à E. coli ou au baculovirus (US-A-4 745 051). On intègre la ou les séquences codantes ou leurs fragments dans le génome du baculovirus (e.g. le baculovirus Autographa californica Nuclear Polyhedrosis Virus AcNPV) et ce dernier est ensuite propagé sur des cellules d'insectes, e.g. Spodoptera frugiperda Sf9 (dépôt ATCC CRL 1711). On peut encore produire les sous-unités dans des cellules eucaryotes telles que des levures (e.g. Saccharomyces cerevisiae) ou cellules de mammifères (e.g. CHO, BHK).

L'invention a aussi pour objet les polypeptides qui seront produits in vitro par ces moyens d'expression, puis éventuellement purifiés selon les techniques classiques. Elle a aussi pour objet les vaccins de sous-unité comprenant au moins un polypeptide tel qu'ainsi obtenu, ou fragment, dans un véhicule ou diluant acceptable sur le plan vétérinaire et éventuellement un adjuvant acceptable sur le plan vétérinaire.

Pour l'expression in vivo en vue de la réalisation de vaccins vivants recombinants, on insère la ou les séquences codantes ou leurs fragments dans un vecteur d'expression approprié dans des conditions permettant l'expression du ou des polypeptides. Comme vecteurs appropriés, on peut utiliser des virus vivants, de préférence capables de se multiplier chez le porc, non pathogènes pour le porc (naturellement non pathogène ou rendu tel), selon les techniques bien connues de l'homme du métier. On pourra notamment utiliser des herpèsvirus du porc tels que le virus de la maladie d'Aujeszky, l'adénovirus porcin, des poxvirus, notamment le virus de la vaccine, l'avipox, le canarypox, le swinepox. On peut aussi utiliser comme vecteurs des ADN plasmidiques (WO-A-90 11092, WO-A-93 19813, WO-A-94 21797, WO-A-95 20660).

L'invention a donc aussi pour objet les vecteurs et les vaccins vivants recombinants ou plasmidiques (vaccins polynucléotidiques ou ADN) ainsi réalisés, les vaccins comprenant en outre un véhicule ou diluant acceptable sur le plan vétérinaire.

Les vaccins selon l'invention (sous-unités, vivants recombinants, plasmidiques) pourront comprendre un ou des principes actifs (antigènes) d'un ou de plusieurs (2 ou 3) des circovirus selon l'invention.

Pour chacun des types de vaccins décrits ci-dessus, la vaccination contre le circovirus porcin est décrite comme pouvant aussi être associée une vaccination contre d'autres pathogènes du porc, en particulier ceux pouvant être associés au syndrome PMWS. Les vaccins divulgués, notamment inactivés, pourront donc comprendre une autre valence correspondant à un autre pathogène du porc. Parmi ces autres pathogènes du porc, on peut citer de préférence le PRRS (Porcine Reproductory and Respiratory Syndrome) (l'homme du métier pourra se reporter à WO-A-93/07898, WO-A-94/18311, FR-A-2 709 966 ; C. Chareyre et al., Proceedings of the 15th IPVS Congress Birmingham, England, 5-9 juillet 1998, p 139 ; incorporés par référence) et/ou Mycoplasma hyopneumoniae (l'homme du métier pourra se reporter à EP-A-597 852, EP-A-550 477, EP-A-571 648, O. Martinon et al., p 157, p 284, p 285 et G. Reynaud et al., p 150 du Proceedings of the 15th IPVS Congress ci-dessus ; incorporés par référence). Parmi les autres valences intéressantes, on peut encore citer Actinobacillus pleuropneumoniae, E. coli et Rhinite atrophique du porc, ou encore maladie d'Aujeszky, peste porcine classique (Hog Cholera), grippe porcine

Est aussi divulguée une méthode permettant d'induire une réponse immunitaire chez le porc vis-à-vis des circovirus décrits. Elle a en particulier pour objet une méthode de vaccination efficace chez le porc.

Cette méthode prévoit l'administration au porc, en une ou plusieurs fois, d'un vaccin supra. Il est aussi possible de combiner plusieurs types de vaccins supra dans un même protocole de vaccination.

Cette méthode prévoit non seulement l'administration aux porcs adultes, mais aussi aux jeunes ou aux femelles gestantes. La vaccination de ces dernières permet de conférer une immunité passive aux nouveau-nés (anticorps maternels).

Il est aussi décrit la possibilité de diagnostiquer la présence des circovirus selon l'invention chez le porc. Elle décrit des tests de diagnostic et méthodes y relatives mettant en oeuvre les réactifs qui vont être décrits ci-après.

La connaissance des séquences des différents circovirus permet de définir des séquences communes qui permettent de produire des réactifs aptes à reconnaître l'ensemble des circovirus porcins connus.

L'homme du métier pourra aussi choisir des fragments des séquences correspondant à des régions présentant peu ou pas d'homologie avec la séquence correspondante du circovirus PK/15 afin de pouvoir effectuer un diagnostic spécifique.

Les alignements de séquences permettent à l'homme du métier de choisir un réactif conforme à ses souhaits.

Un premier réactif consiste dans les séquences d'ADN divulguées ici et leurs fragments, qui seront notamment utilisés comme sondes ou amorces dans des techniques d'hybridation ou de PCR ("Polymerase Chain Reaction") bien connues.

Un deuxième réactif consiste dans les polypeptides codés par ces séquences à partir du virus ou exprimés à l'aide d'un vecteur (voir supra), ou synthétisés par voie chimique selon les techniques classiques de synthèse peptidique.

Un troisième et quatrième réactifs consistent dans des anticorps respectivement polyclonaux et monoclonaux qui pourront être produits selon les techniques usuelles à partir du virus, des polypeptides ou fragments, extraits ou codés par les séquences d'ADN.

Ces deuxième, troisième et quatrième réactifs pourront être utilisés dans une méthode de diagnostic dans laquelle l'on recherche, dans un échantillon de fluide physiologique (sang, plasma, sérum, etc.) ou prélèvement de tissu (ganglions, foie, poumons, reins, etc.) provenant d'un porc à tester, la présence d'un antigène spécifique d'un circovirus selon l'invention, en cherchant à détecter soit l'antigène lui-même, soit des anticorps dirigés contre cet antigène.

Les antigènes et anticorps pourront être utilisés dans toutes les techniques de diagnostic de laboratoire connues.

Toutefois, on préférera les mettre à profit dans des techniques pouvant être mises en oeuvre directement sur le terrain par le vétérinaire, l'éleveur ou le propriétaire de l'animal. L'homme du métier dispose de l'ensemble des techniques de laboratoire et du terrain et est donc parfaitement en mesure de les adapter à l'utilisation de cet antigène et/ou des anticorps comme réactif(s) de diagnostic.

Les techniques de diagnostic qui seront préférentiellement utilisées sont le Western Blot, l'immunofluorescence, l'ELISA et l'immunochromatographie.

En ce qui concerne la mise en oeuvre de méthodes par immunochromatographie, le spécialiste pourra se reporter notamment à Robert F. Zurk et al., Clin. Chem. 31/7, 1144-1150 (1985) ainsi qu'aux brevets ou demandes de brevet WO-A-88/08 534, WO-A-91/12528, EP-A-291 176, EP-A-299 428, EP-A-291 194, EP-A-284 232, US-A-5 120 643, US-A-5 030 558, US-A-5 266 497, US-A-4 740 468, US-A-5 266 497, US-A-4 855 240, US-A-5 451 504, US-A-5 141 850, US-A-5 232 835 et US-A-5 238 652.

Ainsi, l'on cherche de préférence à détecter les anticorps spécifiques dans l'échantillon par test indirect, par compétition ou par déplacement. Pour ce faire, on utilise l'antigène lui-même comme réactif de diagnostic, ou un fragment de cet antigène, conservant la reconnaissance des anticorps. Le marquage peut avantageusement être un marquage à la péroxydase ou un marquage particulaire, de préférence à l'or colloïdal.

On peut aussi chercher à détecter l'antigène lui-même dans l'échantillon à l'aide d'un anticorps marqué spécifique de cet antigène. Le marquage est avantageusement comme décrit ci-dessus.

Par anticorps spécifique de l'antigène utilisable notamment en compétition ou déplacement ou pour la détection de l'antigène lui-même, on entend anticorps monoclonaux et polyclonaux spécifiques de l'antigène, fragments de ces anticorps, de préférence fragments Fab ou F(ab)'₂.

La production d'anticorps, polyclonaux ou monoclonaux, spécifiques de l'antigène conforme à l'invention est également décrite, ces anticorps pouvant être ensuite utilisés notamment comme réactifs de diagnostic pour la détection de l'antigène dans un échantillon de fluide physiologique ou dans un prélèvement de tissu, ou même pour la détection d'anticorps présents dans un tel échantillon ou prélèvement. Les fragments immunologiquement fonctionnels de ces anticorps, en particulier les fragments F(ab) et F(ab)'₂ sont aussi divulgués.

Des anticorps pourront être préparés par les techniques usuelles. On peut notamment se référer à Antibodies, A Laboratory Manual, 1988, Cold Spring Harbor Laboratory, USA ou à J.W. Goding, Monoclonal Antibodies : Principles and Pratice, Academic Press Inc., dont les contenus sont incorporés ici par référence.

On pourra notamment procéder, comme cela est connu en soi, à la fusion de cellules spléniques de souris immunisées par l'antigène ou par au moins l'un de ses fragments, avec des cellules myélomateuses adéquates.

Une préparation, de préférence pure ou partiellement purifiée, ou même brute d'anticorps monoclonaux ou polyclonaux spécifiques de l'antigène, notamment anticorps de souris ou de lapin est également décrite.

Il est également possible de déterminer des épitopes d'intérêt notamment sur la base des séquences d'ADN décrites ici, que ce soient des épitopes d'intérêt vaccinal ou des épitopes d'intérêt en diagnostic. A partir de la séquence d'ADN du génome du circovirus selon l'invention, l'homme du métier est à même de déterminer des épitopes selon les méthodes connues par exemple programme informatique approprié ou PEPSCAN. Les épitopes sont des régions immunodominantes de protéines et sont à ce titre des régions exposées à la surface des protéines. Ils peuvent être donc reconnus par des anticorps et ainsi être particulièrement employés dans le domaine du diagnostic soit pour la préparation d'anticorps à des fins de diagnostic soit pour la réalisation de peptides correspondants utilisables à titre de réactifs de diagnostic.

Au minimum, un épitope est un peptide ayant de 8 à 9 acides aminés. On préférera en général un minimum de 13 à 25 acides aminés.

L'homme du métier est donc en mesure, en utilisant l'une ou plusieurs de ces techniques ainsi que les autres techniques disponibles, de trouver des épitopes pour la mise en oeuvre de peptides ou d'anticorps à des fins de diagnostic.

Est également décrit un kit de diagnostic comportant cet antigène et/ou des anticorps polyclonaux ou monoclonaux spécifiques de cet antigène. Il s'agit en particulier de kits de diagnostic correspondants aux techniques de diagnostic décrites plus haut.

Sont maintenant décrits des exemples de réalisation non limitatifs, pris en référence au dessin, dans lequel :
**Figure 1** **:** séquence d'ADN du génome de la souche Imp 1011-48121
**Figure 2** **:** séquence d'ADN du génome de la souche Imp 1011-48285
**Figure 3** **:** séquence d'ADN du génome de la souche Imp 999
**Figure 4** **:** séquence d'ADN du génome de la souche Imp 1010
**Figure 5** : alignement des 4 séquences selon les figures 1 à 4 avec la séquence **de la** souche PCV PK/15
**Figure 6** **:** séquence d'ADN du génome de la souche Imp 999 telle que définie dans le premier dépôt en France du 3 octobre 1997

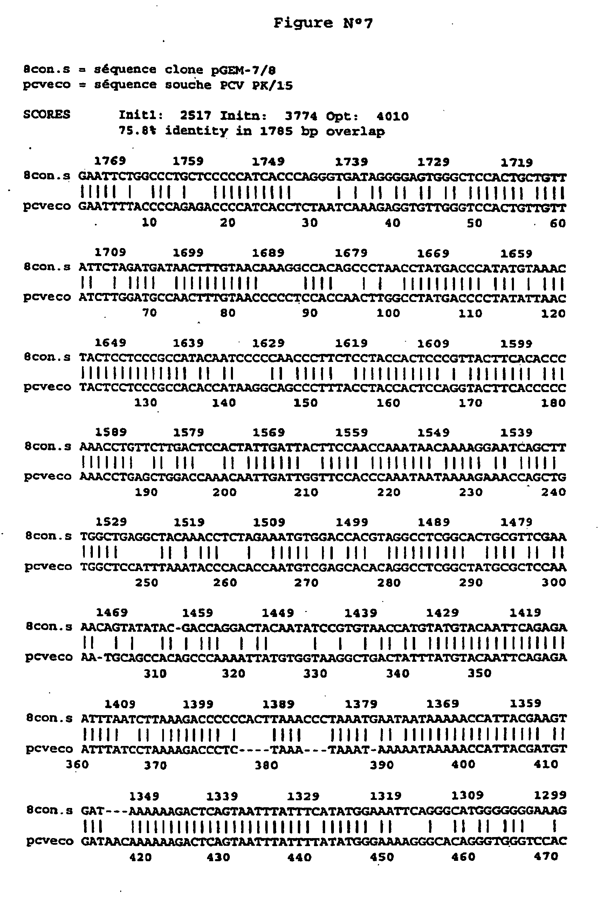

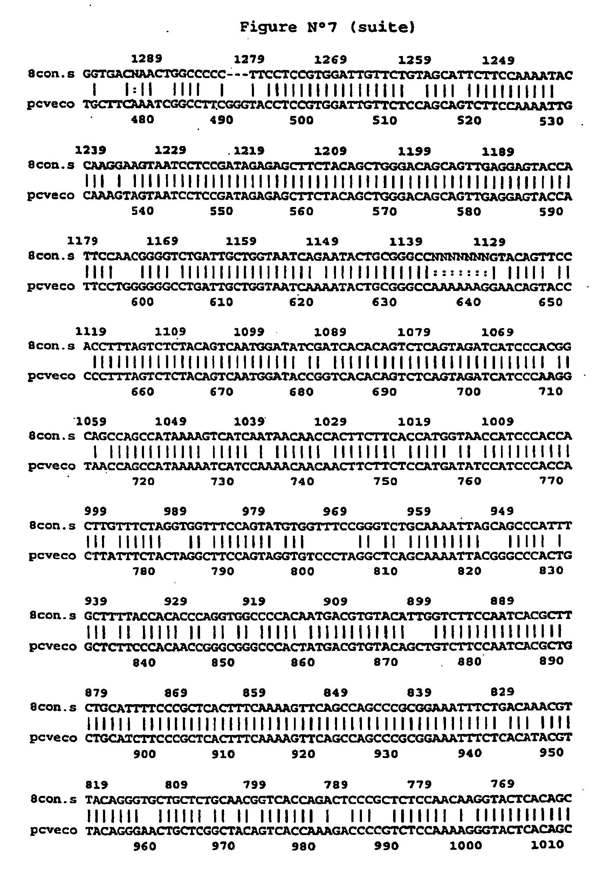

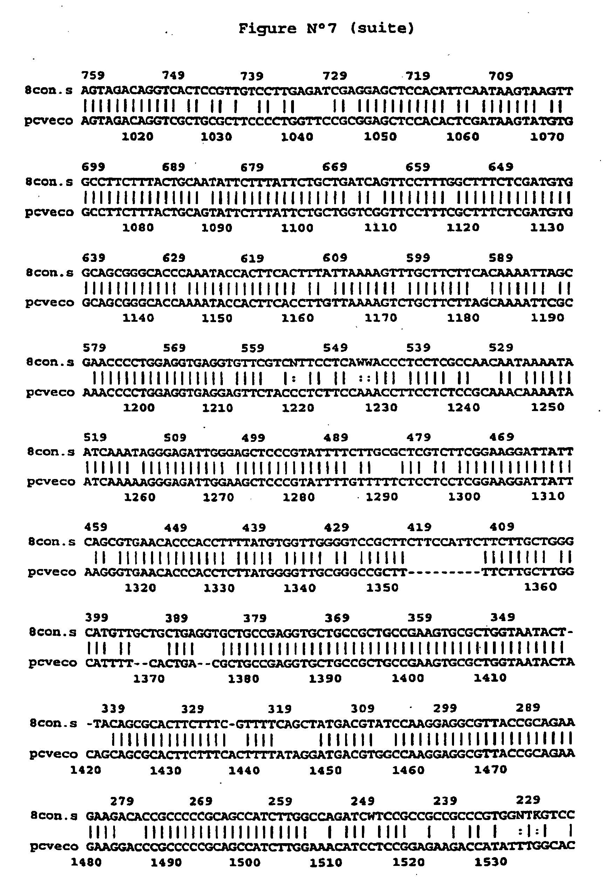

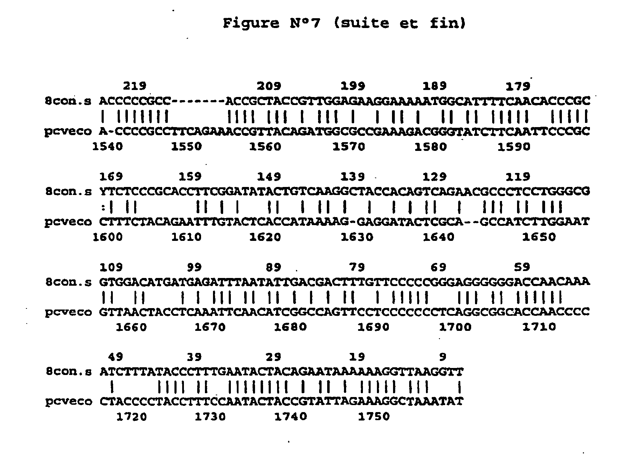

**Figure 7** : Alignements de la séquence de la figure 6 avec la séquence de la souche PK/15

### Liste des séquences SEQ ID

**SEQ ID NO: 1** séquence d'ADN du génome de la souche Imp 1011-48121
**SEQ ID NO: 2** séquence d'ADN du génome de la souche Imp 1011-48285
**SEQ ID NO: 3** séquence d'ADN du génome de la souche Imp 999
**SEQ ID NO: 4** séquence d'ADN du génome de la souche Imp 1010
**SEQ ID NO: 5** séquence d'ADN du génome de la souche PK/15
**SEQ ID NO: 6** séquence d'ADN du génome de la souche Imp 999 telle que définie dans le premier dépôt en France du 3 octobre 1997.

### EXEMPLES

### Exemple 1 : Culture et isolement des souches de circovirus porcins:

Des échantillons de tissus ont été récoltés en France, au Canada et aux USA à partir de poumons et de ganglions lymphatiques de porcelets. Ces porcelets présentaient des signes cliniques typiques du syndrome de dépérissement généralisé de post-sevrage. Pour faciliter l'isolement des virus, les échantillons de tissus ont été congelés à -70°C immédiatement après autopsie.

Pour l'isolement viral, des suspensions contenant environ 15% d'échantillon de tissu ont été préparées dans un milieu minimum contenant des sels d'Earl (EMEM, BioWhittaker UK Ltd., Wokingham, UK), de la pénicilline (100 Ul/ml) et de la streptomycine (100 µg/ml)(milieu MEM-SA), par broyage des tissus avec du sable stérile au moyen d'un mortier et d'un pilon stériles. Cette préparation broyée a été alors reprise dans du MEM-SA, puis centrifugée à 3000 g pendant 30 minutes à + 4°C pour récolter le surnageant.

Préalablement à l'ensemencement des cultures de cellules, un volume de 100 µl de chloroforme a été ajouté à 2 ml de chaque surnageant et mélangé en continu pendant 10 minutes à température ambiante. Ce mélange a alors été transféré dans un tube de microcentrifugeuse, centrifugé à 3000 g pendant 10 minutes, puis le surnageant a été récolté. Ce surnageant a été ensuite utilisé comme inoculum pour les expériences d'isolement viral.

Toutes les études d'isolement viral ont été réalisées sur des cultures de cellules PK/15, connues pour être non contaminées par le circovirus porcin (PCV), les pestivirus, les adénovirus porcins et le parvovirus porcin (Allan G. et al. Pathogenesis of porcine circovirus experimental infections of colostrum-deprived piglets and examination of pig-foetal material. Vet. Microbiol. 1995. 44. 49-64).

L'isolement des circovirus porcins a été réalisé selon la technique suivante:

Des monocouches de cellules PK/15 ont été dissociées par trypsination (avec un mélange trypsine-versène) à partir de cultures confluentes, et reprises en milieu MEM-SA contenant 15% de sérum foetal de veau non contaminé par du pestivirus (= milieu MEM-G) sous une concentration finale d'environ 400,000 cellules par ml. Des fractions aliquotes de 10 ml de cette suspension cellulaire ont alors été mélangées avec des fractions aliquotes de 2 ml des inoculums décrits ci-dessus, et les mélanges finaux ont été aliquotés en volumes de 6 ml dans deux flacons Falcon de 25 cm². Ces cultures ont alors été incubées à +37°C pendant 18 heures en atmosphère contenant 10% de CO₂.

Après incubation, le milieu de culture des monocouches semi-confluentes a été traité avec 300 mM de D-glucosamine (Cat # G48175, Sigma-Aldrich Company Limited, Poole, UK) (Tischr 1. et al., Arch. Virol. 1987 96 39-57), puis l'incubation a été poursuivie pendant une période supplémentaire de 48-72 heures à +37°C. A la suite de cette dernière incubation, l'un des deux Falcons de chaque inoculum a subi 3 cycles successifs de congélation/décongélation. Les cellules PK/15 du Falcon restant ont été traitées avec une solution de trypsine-versène, resuspendues dans 20 ml de milieu MEM-G, puis ensemencées dans des Falcons de 75 cm² à une concentration de 400,000 cellules/ml. Les flacons fraîchement ensemencés ont alors été "surinfectés" par addition de 5 ml du lysat correspondant obtenu après les cycles de congélation/décongélation.

### Exemple 2 : Préparation des échantillons de culture cellulaire pour détection des circovirus porcins par immunofluorescence ou par hybridation in situ.

Un volume de 5 ml de la suspension "surinfectée" a été prélevé et ensemencé dans une boîte de Petri de 55 mm de diamètre contenant une lamelle de verre stérile et dégraissée. Les cultures en flacons et sur lamelles de verre ont été incubées à +37°C et traitées à la glucosamine comme décrit dans l'exemple 1. Les cultures sur lamelles de verre ont été récoltées de 24 à 48 heures après le traitement à la glucosamine et fixées, soit avec de l'acétone pendant 10 minutes à température ambiante, soit avec 10% de formaldéhyde tamponné pendant 4 heures. Suite à cette fixation, toutes les lamelles de verre ont été stockées à -70°C, sur gel de silice, avant leur utilisation pour les études d'hybridation *in situ* et les études de marquage immunocytochimique.

### Exemple 3 : Techniques de détection de séquences PCV par hybridation in situ

L'hybridation *in situ* a été réalisée sur les tissus prélevés sur les porcs malades et fixés au formaldéhyde et également sur les préparations de cultures de cellules inoculées pour l'isolement viral (voir exemple 2) et fixées sur lamelles de verre.

Des sondes génomiques complètes correspondant au circovirus porcin PK/15 (PCV) et au virus de l'anémie infectieuse du poulet (chicken anemia virus = CAV) ont été utilisées. Le plasmide pPCV1, contenant la forme réplicative du génome PCV clonée sous la forme d'un insert unique de 1,7 kilopaires de bases (kpb) (Meehan B. et al. Sequence of porcine circovirus DNA: affinities with plant circoviruses. J. Gen. Virol. 1997. 78. 221-227) a été utilisé comme source d'ADN viral spécifique pour PCV. Un plasmide analogue, pCAA1, contenant la forme réplicative 2,3 kpb du circovirus aviaire CAV a été utilisé comme contrôle négatif. Les stocks de glycérols respectifs de ces deux plasmides ont été utilisés pour la production et la purification des plasmides selon la technique de lyse alcaline (Sambrook J. et al. Molecular cloning : A Laboratory Manual. 2nd Edition. Cold Spring Harbor Laboratory. Cold Spring Harbor. New york. 1989) afin qu'ils servent ensuite de matrices pour la préparation des sondes. Les sondes circovirus représentatives des génomes complets du PCV et de CAV ont été produites à partir des plasmides purifiés décrits ci-dessus (1µg pour chaque sonde) et d'amorces hexanucléotidiques au hasard en utilisant un kit commercial de marquage non radioactif ("DIG DNA labelling kit" , Boehringer Mannheim, Lewes, UK) selon les recommandations du fournisseur.

Les sondes marquées à la digoxigénine ont été reprises sous un volume de 50-100 pl d'eau stérile avant leur utilisation pour l'hybridation *in situ*.

Les échantillons de tissus de porcs malades, inclus dans la paraffine et fixés au formaldéhyde, ainsi que les préparations de cultures de cellules infectées, fixées au formaldéhyde, ont été préparées pour la détection des acides nucléiques PCV selon la technique suivante :

Des sections de 5 pm d'épaisseur ont été découpées à partir des blocs de tissus inclus dans la paraffine, déparaffinés, puis réhydratés dans des solutions successives d'alcool à concentration décroissante. Les sections de tissus et les cultures de cellules fixées au formaldéhyde ont été incubées respectivement pendant 15 minutes et 5 minutes à +37° C dans une solution de protéinase K à 0,5% en tampon Tris-HCl 0,05M, EDTA 5 mM (pH 7,6). Les lames ont été alors placées dans une solution de glycine à 1 % en eau distillée autoclavée, pendant 30 secondes, lavées deux fois avec un tampon PBS (phosphate buffer saline) 0,01 M (pH 7,2), et enfin lavées pendant 5 minutes en eau distillée stérile. Elles ont été finalement séchées à l'air libre et mises en contact avec les sondes.

Chaque préparation tissu/sonde a été recouverte avec une lamelle propre et dégraissée, puis placée dans un four à +90°C pendant 10 minutes, mise ensuite en contact avec un bloc de glace pendant 1 minute, et enfin incubée pendant 18 heures à + 37 ° C. Les préparations ont été ensuite immergées brièvement dans un tampon sel de sodium-citrate (SSC) 2X (pH 7,0) pour éliminer les lamelles protectrices, puis lavées 2 fois pendant 5 minutes en tampon SSC 2X et enfin lavées 2 fois pendant 5 minutes en tampon PBS.

Après ces lavages, les préparations ont été immergées dans une solution d'acide maléique 0,1 M, NaCl 0,15 M (pH 7,5) (tampon maléique) pendant 10 minutes, puis incubées dans une solution de 1% de réactif bloquant (Cat # 1096176, Boehringer Mannheim UK, Lewis, East Sussex, UK) en tampon maléique pendant 20 minutes à + 37 °C.

Les préparations ont alors été incubées avec une solution au 1/260 d'un anticorps monoclonal anti-digoxigénine (Boehringer Mannheim), dilué en tampon bloquant, pendant 1 heure à + 37 ° C, lavées en PBS et enfin incubées avec un anticorps biotinylé anti-immunoglobuline de souris pendant 30 minutes à + 37 ° C. Les préparations ont été lavées dans du PBS et l'activité péroxydase endogène a été bloquée par un traitement avec une solution de péroxyde d'hydrogène à 0,5% en PBS pendant 20 minutes à température ambiante. Les préparations ont été lavées une nouvelle fois dans du PBS et traitées avec un substrat 3-amino-9-diéthylcarbazole (AEC) (Cambridge Bioscience, Cambridge, UK) préparé extemporanément.

Après un dernier lavage à l'eau de ville, les préparations ont été contre-colorées avec de l'hématoxyline, "bleuies" sous eau de ville, et montées sous lamelles microscopiques avec un liquide de montage (GVA Mount, Cambridge Bioscience, Cambridge, UK). Les contrôles d'expérience ont inclus l'utilisation d'une sonde négative non pertinente (CAV) et d'une sonde positive (PCV) sur des échantillons provenant de porcs malades et de porcs non malades.

### Exemple 4 : Technique de détection du PCV par immunofluorescence

Le criblage initial de toutes les préparations de culture cellulaire fixées à l'acétone a été réalisé par une technique d'immunofluorescence indirecte (IFI) utilisant une dilution au 1/100 d'un pool de sérums de porcs adultes. Ce pool de sérums comprend des sérums de 26 truies adultes d'Irlande du Nord et est connu pour contenir des anticorps contre une grande variété de virus porcins, y compris PCV : parvovirus porcin, adénovirus porcin, et virus PRRS. La technique IFI a été réalisée par un contact du sérum (dilué en PBS) avec les cultures cellulaires pendant une heure à + 37°C, suivi de deux lavages dans du PBS. Les cultures de cellules sont alors colorées avec une dilution au 1/80 en PBS d'un anticorps de lapin anti-immunoglobuline de porc conjugué à de l'isothiocyanate de fluorescéine pendant une heure, puis lavées dans du PBS et montées en tampon glycérol préalablement à l'observation microscopique sous éclairage ultra-violet.

### Exemple 5 : Résultats de l'hybridation in situ sur les tissus de porcs malades

*L'hybridation in situ*, utilisant une sonde génomique PCV, réalisée sur des tissus prélevés sur des porcelets français, canadiens et californiens présentant des lésions de dépérissement généralisé et fixés au formaldéhyde, a révélé la présence d'acides nucléiques PCV associés aux lésions, dans plusieurs des lésions étudiées. Aucun signal n'a été observé lorsque la sonde génomique PCV a été utilisée sur des tissus prélevés sur des porcs non malades ou lorsque la sonde CAV a été utilisée sur les tissus de porcs malades. La présence d'acide nucléique PCV a été identifiée dans le cytoplasme et le noyau de nombreuses cellules mononucléaires infiltrant les lésions dans les poumons des porcelets californiens. La présence d'acide nucléique PCV a également été mise en évidence dans les pneumocytes, les cellules épithéliales bronchiques et bronchiolaires, et dans les cellules endothéliales des petites artérioles, veinules et vaisseaux lymphatiques.

Chez les porcs malades français, la présence d'acide nucléique PCV a été détectée dans le cytoplasme de nombreux lymphocytes folliculaires et dans les cellules mononucléaires intrasinusoïdales des ganglions lymphatiques. L'acide nucléique PCV a également été détecté dans des syncytia occasionnels. En fonction de ces résultats de détection, des échantillons de poumons de porcs californiens, de ganglions lymphatiques mésentériques de porcs français, et d'organes de porcs canadiens ont été choisis aux fins d'isolement des nouvelles souches de circovirus porcin.

### Exemple 6 : Résultats de la culture cellulaire des nouvelles souches de circovirus porcin et détection par immunofluorescence

Aucun effet cytopathique (ECP) n'a été observé dans les cultures de cellules inoculées avec les échantillons prélevés sur les porcelets francais (souche Imp.1008), californiens (souche Imp.999) et canadiens (souche Imp.1010) montrant des signes cliniques du syndrome du dépérissement généralisé. Cependant, l'immuno-marquage des préparations provenant des cultures de cellules inoculées, après fixation à l'acétone et avec un pool de sérums polyclonaux de porcs, a révélé une fluorescence nucléaire chez de nombreuses cellules dans les cultures inoculées à partir des poumons de porcelets californiens (souche Imp.999), à partir des ganglions lymphatiques médiastinaux des porcelets français (souche Imp.1008), et à partir d'organes des porcelets canadiens (souche Imp.1010).

### Exemple 7 : Extraction de l'ADN génomique des circovirus porcins

Les formes réplicatives des nouvelles souches de circovirus porcin (PCV) ont été préparées à partir de cultures de cellules PK/15 infectées (voir exemple 1) (10 Falcons de 75 cm²) récoltées après 72-76 heures d'incubation et traitées à la glucosamine, comme décrit pour le clonage de la forme réplicative du CAV (Todd. D. et al. Dot blot hybridization assay for chicken anemia agent using a cloned DNA probe. J. Clin. Microbiol. 1991. 29. 933-939). L'ADN double brin de ces formes réplicatives a été extrait selon une modification de la technique de Hirt (Hirt B. Selective extraction of polyoma virus DNA from infected cell cultures. J. Mol. Biol. 1967. 36. 365-369), comme décrit par Molitor (Molitor T.W. et al. Porcine parvovirus DNA: characterization of the genomic and replicative form DNA of two virus isolates. Virology. 1984. 137. 241-254).

### Exemple 8 : Carte de restriction de la forme réplicative du génome de la souche Imp.999 de circovirus porcin.

L'ADN (1-5 µg) extrait selon la technique de Hirt a été traité par la nucléase S1 (Amersham) selon les recommandations du fournisseur, puis cet ADN a été digéré par différentes enzymes de restriction (Boehringer Mannheim, Lewis, East Sussex, UK) et les produits de la digestion ont été séparés par électrophorèse sur gel d'agarose à 1,5% en présence de bromure d'éthidium comme décrit par Todd et al. (Purification and biochemical characterization of chicken anemia agent. J. Gen. Virol. 1990. 71. 819-823). - L'ADN extrait des cultures de la souche Imp.999 possède un site unique EcoRI, 2 sites Sacl et ne possède pas de site Pstl. Ce profil de restriction est donc différent du profil de restriction présenté par la souche PCV PK/15 (Meehan B. et al. Sequence of porcine circovirus DNA: affinities with plant circoviruses. 1997. 78. 221-227) qui possède au contraire un site Pstl et ne possède pas de site EcoRI.

### Exemple 9 : Clonage du génome de la souche Imp.999 de circovirus porcin

Le fragment de restriction d'environ 1,8 kpb généré par digestion de la forme réplicative double brin de la souche PCV Imp.999 avec l'enzyme de restriction EcoRI a été isolé après électrophorèse sur gel d'agarose à 1,5% (voir exemple 3) en utilisant un kit commercial Qiagen (QIAEXII Gel Extraction Kit, Cat # 20021,QIAGEN Ltd., Crawley, West Sussex, UK). Ce fragment de restriction EcoRI-EcoRI a été ensuite ligaturé avec le vecteur pGEM-7 (Promega, Medical Supply Company, Dublin, Ireland), préalablement digéré par les mêmes enzymes de restriction et déphosphorylé, en suivant les techniques standards de clonage (Sambrook J. et al. Molecular cloning : A Laboratory Manual. 2nd Edition. Cold Spring Harbor Laboratory. Cold Spring Harbor. New york. 1989). Les plasmides obtenus ont été transformés dans une souche hôte *Escherichia coli* JM109 (Stratagene, La Jolla, USA) selon les techniques standards. Le fragment de restriction EcoRI-EcoRI de la souche PCV Imp.999 a également été cloné dans le site EcoRI du vecteur pBlueScript SK+ (Stratagene Inc. La Jolla, USA). Parmi les clones obtenus pour chaque souche hôte, au moins 2 clones contenant les fragments de la taille attendue ont été sélectionnés. Les clones obtenus ont alors été cultivés et les plasmides contenant le génome complet de la souche Imp.999 ont été purifiés en petit volume (2 ml) ou en grand volume (250 ml) selon les techniques standards de préparation et de purification des plasmides.

### Exemple 10 : Séquençage de l'ADN génomique (forme réplicative double brin) de la souche PCV Imp.999.

La séquence nucléotidique de 2 clones EcoRI Imp.999 (clones pGEM-7/2 et pGEM-7/8) a été déterminée selon la technique des didéoxynucléotides de Sanger en utilisant le kit de séquençage "AmpliTaq DNA polymerase FS" (Cat # 402079 PE Applied Biosystems, Warrington, UK) et un appareil de séquençage automatique Applied BioSystems ABI373A selon les recommandations du fournisseur. Les réactions de séquençage initiales ont été faites avec les primers universels M13 "forward" et "reverse". Les réactions de séquençage suivantes ont été générées selon la technique de "marche sur l'ADN". Les oligonucléotides nécessaires à ces séquençages ultérieurs ont été synthétisés par Life Technologies (inchinnan Business Park, Paisley, UK).

Les séquences générées ont été assemblées et analysées au moyen du logiciel MacDNASIS version 3.2. (Cat # 22020101, Appligene, Durham, UK). Les différents cadres ouverts de lecture ont été analysés au moyen de l'algorithme BLAST disponible sur le serveur du "National Center for Biotechnology Information (NCBI, Bethesda, MD, USA).

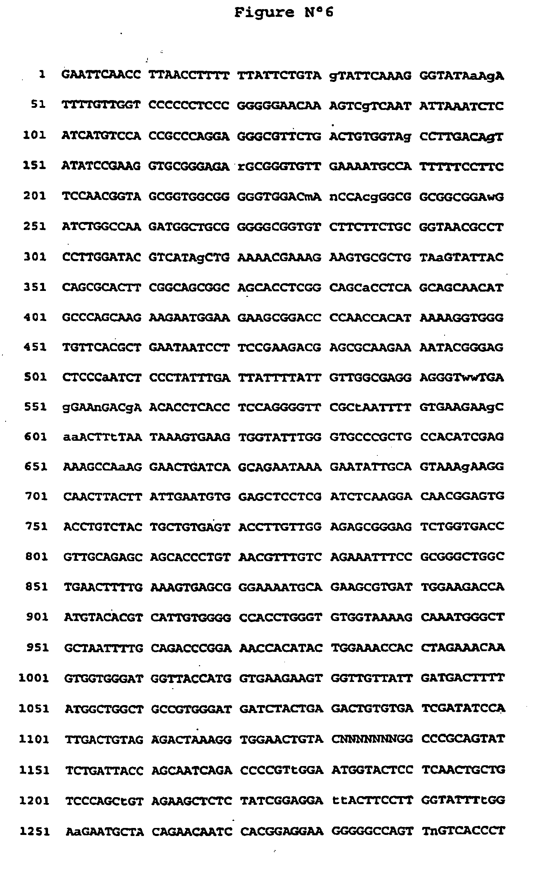

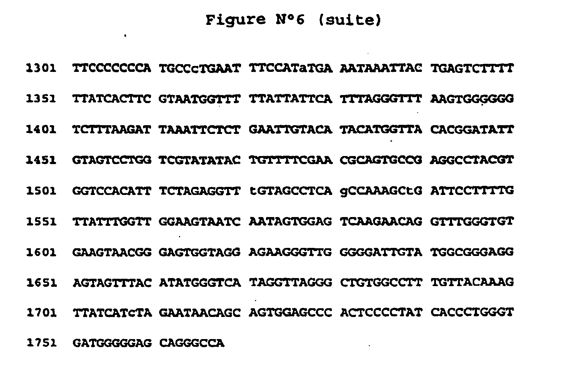

La séquence complète (fragment EcoRI-EcoRI) obtenue initialement à partir du clone pGEM-7/8 (SEQ ID NO : 6) est présentée sur la figure N°6. Elle débute arbitrairement après le G du site EcoRI et présente quelques incertitudes sur le plan des nucléotidiques.

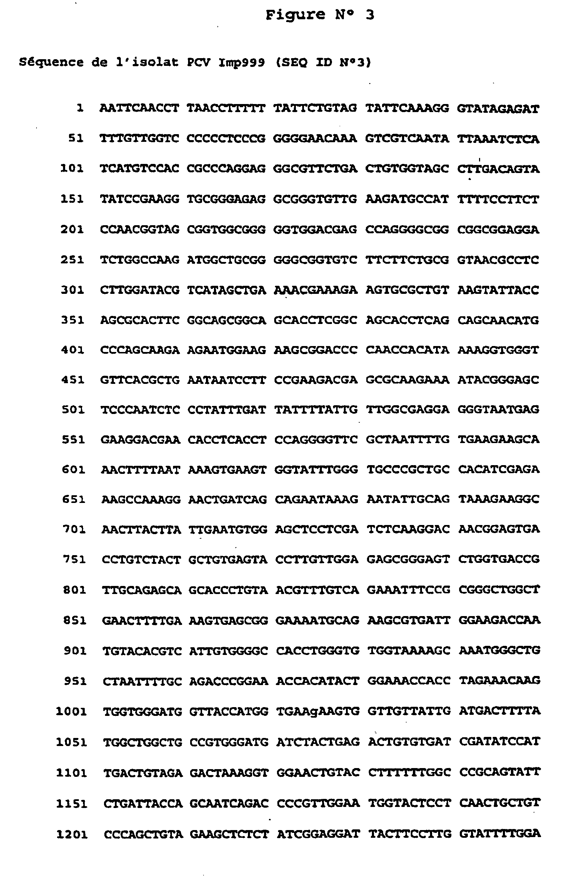

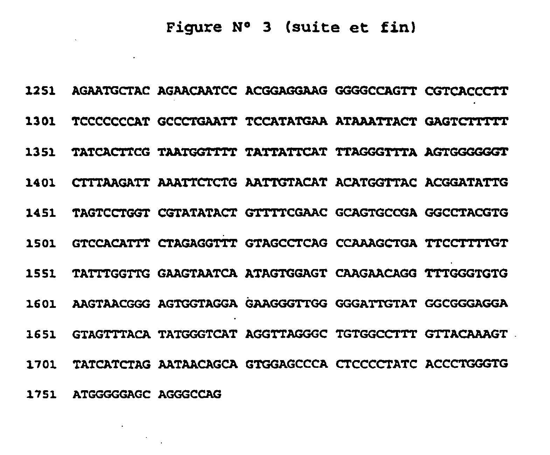

Le séquençage a ensuite été optimisé et la SEQ ID NO : 3 (Figure 3) donne la séquence totale de cette souche, que l'on a fait débuté arbitrairement au début du site EcoRI, soit le G comme premier nucléotide.

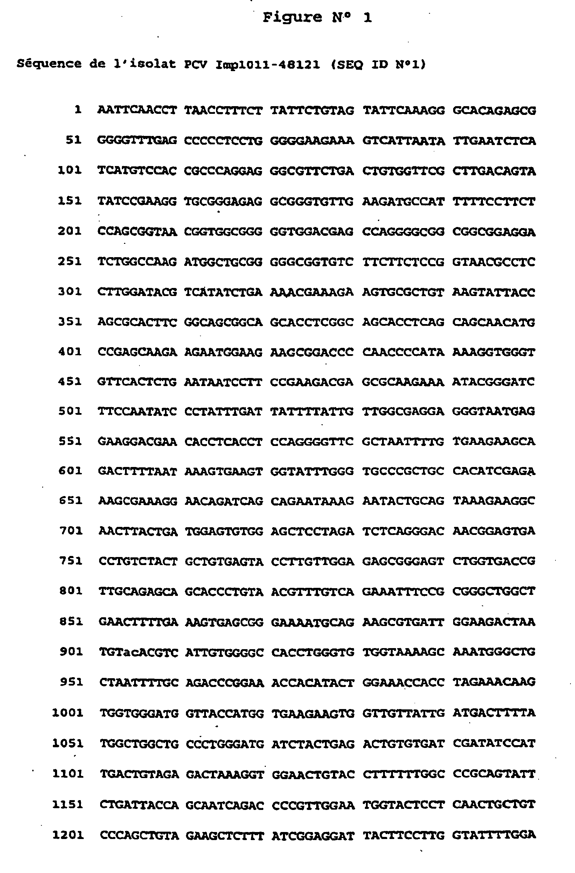

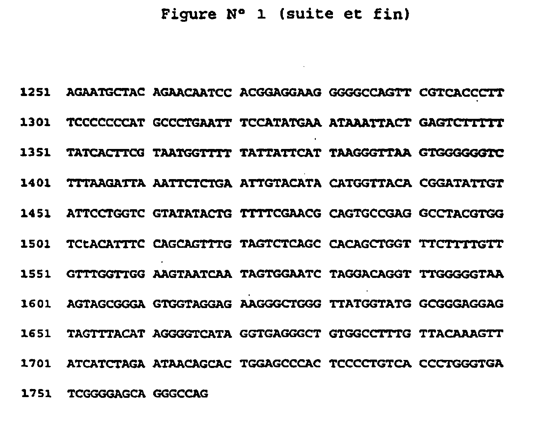

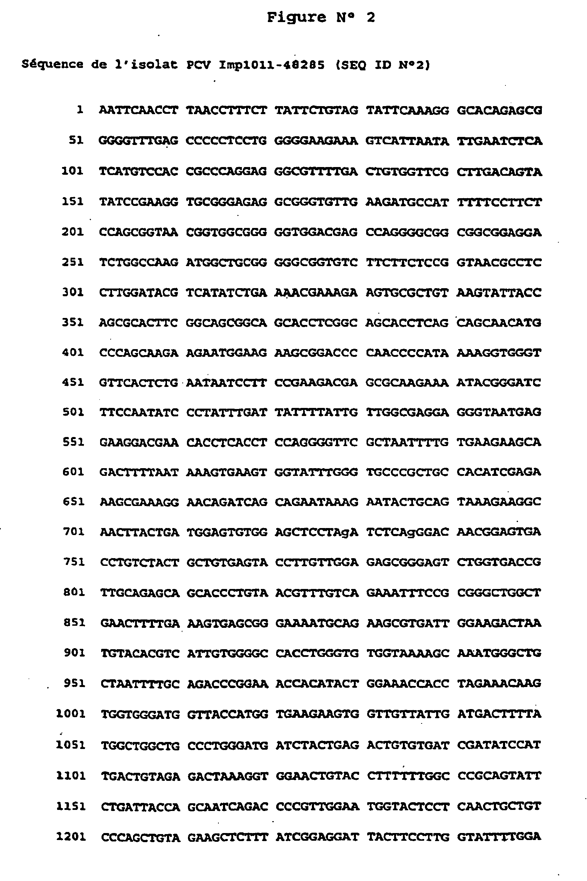

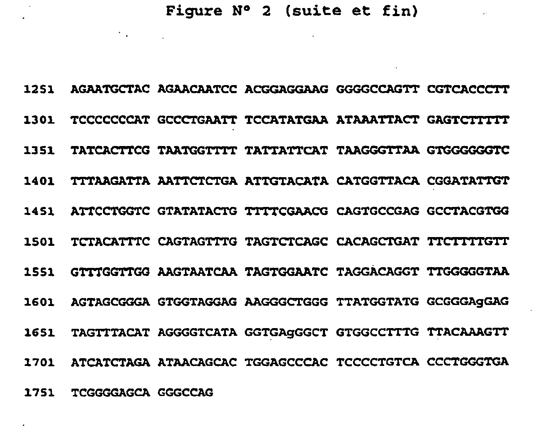

On a procédé d'une manière similaire pour l'obtention de la séquence des trois autres isolats selon l'invention (voir SEQ ID NO : 1, 2 et 4 et figures 1, 2 et 4).

La taille du génome de ces quatre souches est :

| | |
|---|---|
| Imp 1011-48121 | 1767 nucléotides |
| Imp 1011-48286 | 1767 nucléotides |
| Imp 999 | 1768 nucléotides |
| Imp 1010 | 1768 nucléotides |

### Exemple 11 : Analyse de la séquence de la souche PCV Imp.999.

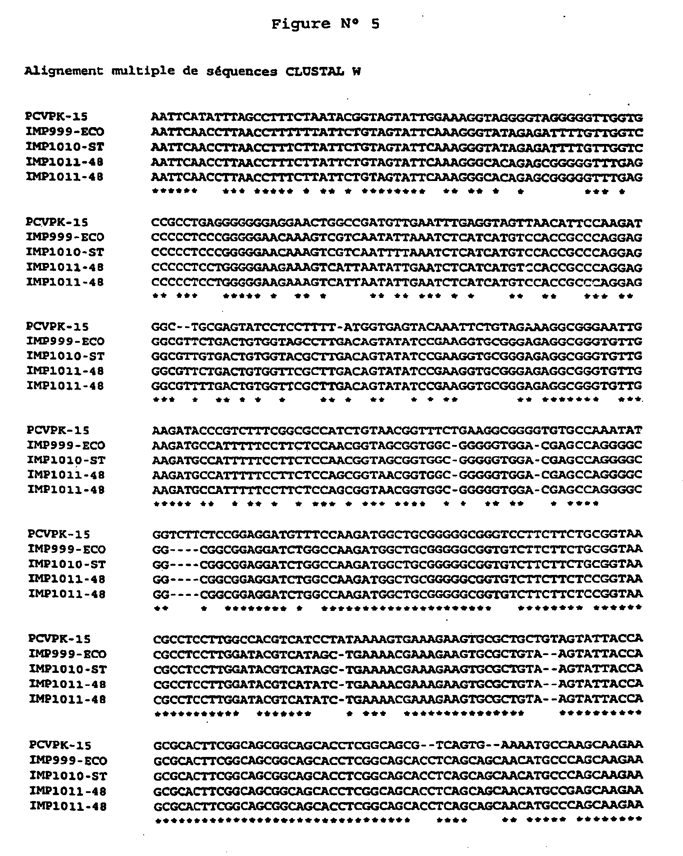

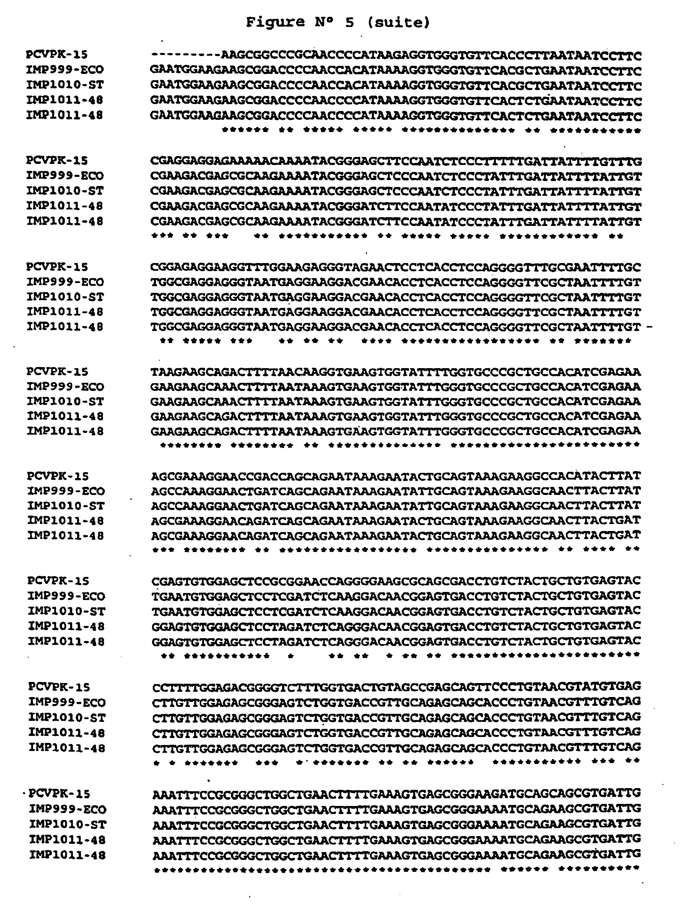

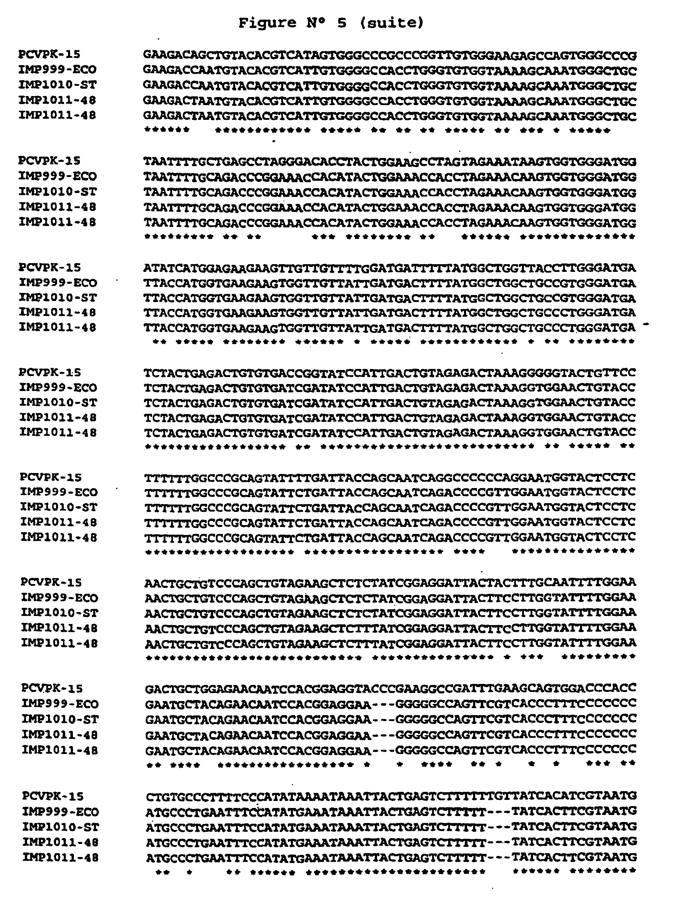

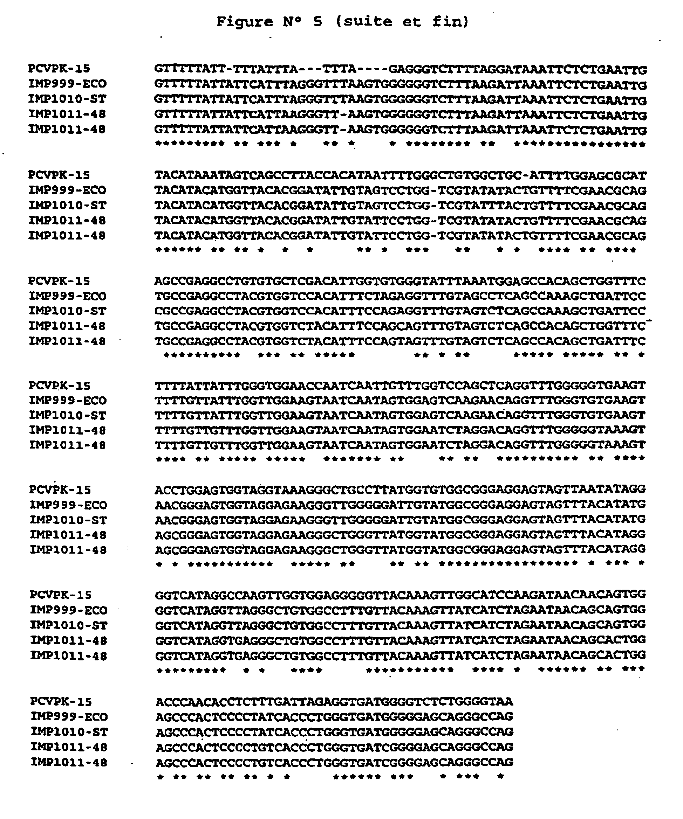

Lorsque la séquence générée à partir de la souche Imp.999 a été utilisée pour une recherche d'homologie vis-à-vis des séquences contenues dans la banque de données GenBank, la seule homologie significative qui ait été détectée est une homologie d'environ 76 % (au niveau acide nucléique) avec la séquence de la souche PK/15 (Numéros d'accès Y09921 et U49186) (voir figure N°5).

Au niveau acides aminés, la recherche d'homologie de la traduction des séquences dans les 6 phases avec les banques de données (algorithme BLAST X sur le serveur NCBI) a permis de mettre en évidence une homologie de 94 % avec le cadre ouvert de lecture correspondant à la réplicase théorique du virus BBTV similaire aux circovirus de plantes (numéro d'identification GenBank 1841515) codée par la séquence GenBank U49186.

Aucune autre séquence contenue dans les banques de données ne montre d'homologie significative avec la séquence générée à partir de la souche PCV Imp.999.

L'analyse des séquences obtenues à partir de la souche Imp.999 cultivée à partir de lésions prélevées sur des porcelets californiens présentant des signes cliniques du syndrome de dépérissement généralisé montre clairement que cet isolat viral est une nouvelle souche de circovirus porcin.

### Exemple 12 : Analyse comparative des séquences

L'alignement des séquences nucléotidiques des 4 nouvelles souches PCV a été fait avec la séquence de la souche PCV PK/15 (figure 5). Une matrice d'homologie prenant en compte les quatre nouvelles souches et la souche antérieure PK/15 a été réalisée. Les résultats sont les suivants :
1 : Imp 1011-48121
2 : Imp 1011-48285
3 : Imp 999
4 : Imp 1010
5 : PK/15

| | **1** | **2** | **3** | **4** | **5** |
|---|---|---|---|---|---|
| 1 | 1,0000 | 0,9977 | 0,9615 | 0,9621 | 0,7600 |
| 2 | | 1,0000 | 0,9621 | 0,9632 | 0,7594 |
| 3 | | | 1,0000 | 0,9949 | 0,7560 |
| 4 | | | | 1,0000 | 0,7566 |
| 5 | | | | | 1,0000 |

L'homologie entre les deux souches françaises Imp 1011-48121 et Imp 1011-48285 est supérieure à 99 % (0,9977).

L'homologie entre les deux souches nord-américaines Imp 999 et Imp 1010 est aussi supérieure à 99 % (0,9949). L'homologie entre souches françaises et souches nord-américaines est un peu supérieure à 96 %.

L'homologie de toutes ces souches avec PK/15 tombe à une valeur comprise entre 75 et 76 %.

On en déduit que les souches selon l'invention sont représentatives d'un nouveau type de circovirus porcin, distinct du type représenté par la souche PK/15. Ce nouveau type, isolé de porcs présentant le syndrome PMWS, est dénommé circovirus porcin de type II, la PK/15 représentant le type I. Les souches appartenant à ce type Il présentent une remarquable homogénéité de séquence nucléotidique, alors même qu'elles ont été isolées dans des régions géographiques très éloignées.

### Exemple 13 : Analyse des protéines codées par le génome des nouvelles souches PCV.

La séquence nucléotidique de l'isolat Imp. 1010 a été considérée comme représentative des autres souches de circovirus associées au syndrome de dépérissement généralisé. Cette séquence a été analysée plus en détail à l'aide de l'algorithme BLASTX (Altschul et al. J. Mol. Biol. 1990. 215. 403-410) et d'une combinaison de programmes de l'ensemble de logiciels MacVector 6.0 (Oxford Molecular Group, Oxford OX4 4GA, UK). Il a été possible de détecter 13 cadres ouverts de lecture (ou COLs) d'une taille supérieure à 20 acides aminés sur cette séquence (génome circulaire). Ces 13 COLs sont les suivants :

| Nom | Début | Fin | Brin | Taille du COL (nucléotides (nt)) | Taille protéine (acides aminés (aa)) |
|---|---|---|---|---|---|
| COL1 | 103 | 210 | sens | 108 nt | 35 aa |
| COL2 | 1180 | 1317 | sens | 138 nt | 45 aa |
| COL3 | 1363 | 1524 | sens | 162 nt | 53 aa |
| COL4 | 398 | 1342 | sens | 945 nt | 314 aa |
| COL5 | 900 | 1079 | sens | 180 nt | 59 aa |
| COL6 | 1254 | 1334 | sens | 81 nt | 26 aa |
| COL7 | 1018 | 704 | antisens | 315 nt | 104 aa |
| COL8 | 439 | 311 | antisens | 129 nt | 42 aa |
| COL9 | 190 | 101 | antisens | 90 nt | 29 aa |
| COL10 | 912 | 733 | antisens | 180 nt | 59 aa |
| COL11 | 645 | 565 | antisens | 81 nt | 26 aa |
| COL12 | 1100 | 1035 | antisens | 66 nt | 21 aa |
| COL13 | 314 | 1381 | antisens | 702 nt | 213 aa |

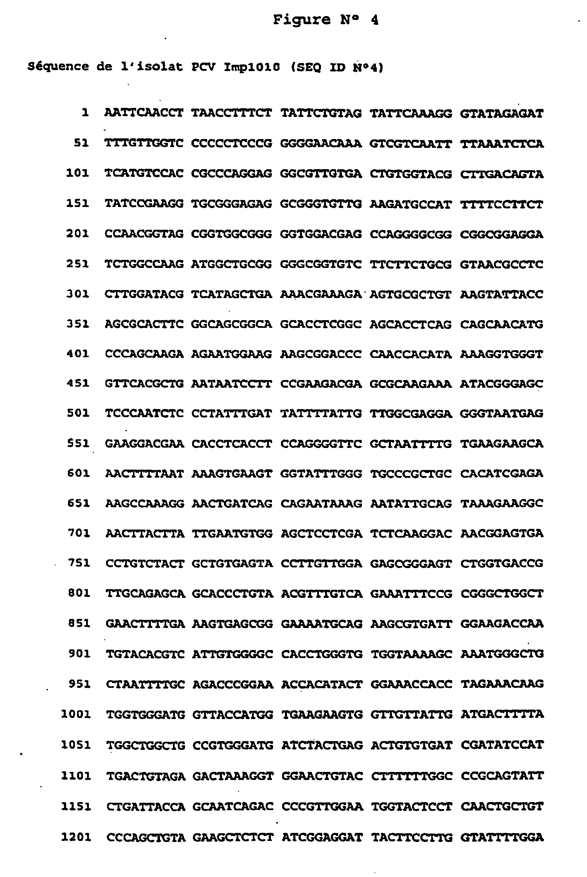

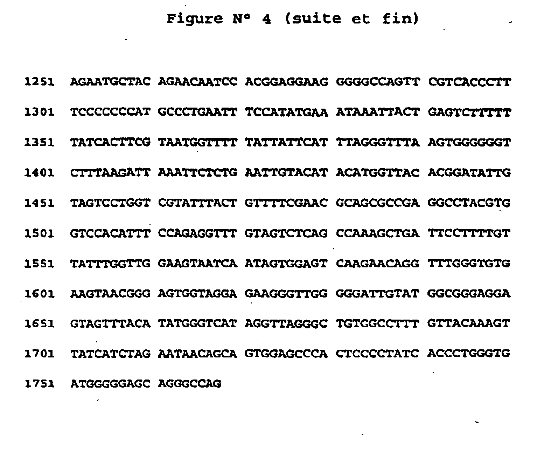

Les positions de début et de fin de chaque COL se réfèrent à la séquence présentée sur la figure N°4 (SEQ ID N° 4), du génome de la souche 1010. Les limites des COLs 1 à 13 sont identiques pour la souche 999. Elles le sont aussi pour les souches 1011-48121 et 1011-48285, sauf pour les COLs 3 et 13 :

| | |
|---|---|
| COL3 | 1432-1539, sens, 108 nt, 36aa |
| COL13 | 314-1377, antisens, 705 nt, 234 aa. |

Parmi ces 13 COLs, 4 présentent une homologie significative avec des COLs analogues situés sur le génome du virus cloné PCV PK-15. Chacun des cadres ouverts de lecture présents sur le génome de tous les isolats de circovirus associés au syndrome de dépérissement généralisé a été analysé. Ces 4 COLs sont les suivants :

| Nom | Début | Fin | Bdn | Taille du COL (nt) | Taille protéine (acides aminés) | Masse moléculaire |
|---|---|---|---|---|---|---|
| COL4 | 398 | 1342 | sens | 945 nt | 314 aa | 37,7 kDa |
| COL7 | 1018 | 704 | antisens | 315 nt | 104 aa | 11,8 kDa |
| COL10 | 912 | 733 | antisens | 180 nt | 59 aa | 6,5 kDa |
| COL13 | 314 | 1381 | antisens | 702 nt | 233 aa | 27,8 kDa |

Les positions de début et de fin de chaque COL se réfèrent à la séquence présentée sur la figure N ° 4 (SEQ ID N ° 4). La taille du COL (en nucléotides = nt) inclut le codon stop.

La comparaison entre l'organisation génomique des isolats PCV Imp. 1010 et PCV PK-15 a permis l'identification de 4 COLs conservés dans le génome des deux virus. Le tableau ci-dessous présente les degrés d'homologie observés:

| **COL Imp.1010/COL PCV PK-15** | **Pourcentage d'homologie** |
|---|---|
| COL4/COL1 | 86 % |
| COL13/CO12 | 66,4 % |
| COL7/COL3 | 61,5 % (au niveau du recouvrement (104 aa) |
| COL10/COL4 | 83 % (au niveau du recouvrement (59 aa) |

La plus grande identité de séquence a été observée entre le COL4 Imp. 1010 et le COL1 PK-15 (86% d'homologie). Ceci était attendu dans la mesure où cette protéine est probablement impliquée dans la réplication de l'ADN viral et est essentielle pour la réplication virale (Meehan et al. J. Gen. Virol. 1997. 78. 221-227; Mankertz et al. J. Gen. Virol. 1998. 79. 381-384).

L'identité de séquence entre le COL13 Imp. 1010 et le COL2 PK-15 est moins forte (66,4% d'homologie), mais chacun de ces deux COLs présente bien une région basique N-terminale très conservée, qui est identique à la région N-terminale de la protéine structurale majeure du circovirus aviaire CAV (Meehan et al. Arch. Virol. 1992. 124. 301-319). De plus grandes différences sont observées entre COL7 Imp. 1010 et COL3 PK-15 et entre COL10 Imp. 1010 et COL4 PK-15. Dans chaque cas, il existe une délétion de la région C-terminale des COL7 et COL10 de l'isolat Imp. 1010 lorsqu'on les compare aux COL3 et COL4 de PCV PK-15. La plus haute homologie de séquence est observée au niveau des régions N-terminales de COL7/COL3 (61,5% d'homologie au niveau du recouvrement) et de COL10/COL4 (83% d'homologie au niveau du recouvrement).

Il apparaît que l'organisation génomique du circovirus porcin est assez complexe suite à l'extrême compacité de son génome. La protéine structurale majeure est probablement issue d'un épissage entre plusieurs cadres de lecture situés sur le même brin du génome du circovirus porcin. On peut donc considérer que tout cadre ouvert de lecture (COL1 à COL13) tel que décrit dans le tableau ci-dessus, peut représenter tout ou partie d'une protéine antigénique codée par le circovirus porcin de type Il et est donc potentiellement un antigène utilisable pour le diagnostic spécifique et/ou pour la vaccination. L'invention concerne donc toute protéine comprenant au moins un de ces COLs. De préférence, l'invention concerne une protéine formée essentiellement par les COL4, COL7, COL10 ou COL13.

### Exemple 14 : Caractère infectieux du génome PCV cloné à partir des nouvelles souches.

Le plasmide pGEM-7/8 contenant le génome complet (forme réplicative) de l'isolat Imp.999 a été transfecté dans des cellules PK/15 selon la technique décrite par Meehan B. et al. (Characterization of viral DNAs from cells infected with chicken anemia agent : sequence analysis of the cloned replicative form and transfection capabilities of cloned genome fragments. Arch. Virol. 1992. 124. 301-319). L'analyse par immunofluorescence (voir exemple 4) réalisée sur le premier passage après transfection sur cellules PK/15 non contaminées a montré que le plasmide du clone pGEM7/8 était capable d'induire la production de virus PCV infectieux. La disponibilité d'un clone contenant un matériel génétique PCV infectieux permet toute manipulation utile sur le génome viral afin de produire des virus PCV modifiés (soit atténués chez le porc, soit défectifs) utilisables pour la production de vaccins atténués ou recombinés, ou pour la production d'antigènes pour des trousses de diagnostic.

### Exemple 15 : Production des antigènes PCV par culture in vitro

La culture des cellules PK/15 non contaminées et la multiplication virale sont réalisées selon les mêmes modalités qu'à l'exemple 1. Les cellules infectées sont récoltées après trypsination après 4 jours d'incubation à 37 ° C et numérées. Le passage suivant est inoculé avec 400 000 cellules infectées par ml.

### Exemple 16 : Inactivation des antigènes viraux

En fin de culture virale, les cellules infectées sont récoltées et lysées par ultrasons (Branson Sonifier) ou à l'aide d'un broyeur colloïdal de type rotor-stator (UltraTurrax, IKA). La suspension est ensuite centrifugée à 3700 g pendant 30 minutes. La suspension virale est inactivée par 0,1 % d'éthylène imine pendant 18 heures à + 37°C ou par 0,5 % de bêta-propiolactone pendant 24 heures à +28°C. Si le titre du virus avant inactivation est insuffisant, la suspension virale est concentrée par ultrafiltration en utilisant une membrane avec un seuil de coupure de 300 kDa (Millipore PTMK300). La suspension virale inactivée est conservée à + 5°C.

### Exemple 17 : Préparation du vaccin sous forme d'émulsion à base d'huile minérale.

Le vaccin est préparé selon la formule suivante :
- suspension de circovirus porcin inactivé : 250 ml
- Montanide® ISA 70 (SEPPIC): 750 ml

La phase aqueuse et la phase huileuse sont stérilisées séparément par filtration. L'émulsion est préparée par mélange et homogénéisation des ingrédients à l'aide d'un émulseur à turbine Silverson.

Une dose de vaccin contient environ 10^{7,5} DICT50. Le volume d'une dose de vaccin est de 0,5 ml pour administration par voie intradermique, et de 2 ml pour administration par voie intramusculaire.

### Exemple 18 : Préparation du vaccin sous forme d'émulsion à base d'huile métabolisable.

Le vaccin est préparé selon la formule suivante :
- suspension de circovirus porcin inactivé : 200 ml
- Dehymuls HRE 7 (Henkel) : 60 ml
- Radia 7204 (Oleofina) : 740 ml

La phase aqueuse et la phase huileuse sont stérilisées séparément par filtration. L'émulsion est préparée par mélange et homogénéisation des ingrédients à l'aide d'un émulseur à turbine Silverson. -

Une dose de vaccin contient environ 10^{7,5} DICT50. Le volume d'une dose de vaccin est de 2 ml pour administration par voie intramusculaire.

### Exemple 19 : Résultats d'immunofluorescence indirecte vis-à-vis des souches de virus PCV US et française et du contaminant PK/15 avec un sérum hyperimmun (PCV-T), un panel d'anticorps monoclonaux F99, préparés à partir de PK/15 et un sérum hyperimmun préparé à partir de la souche canadienne (PCV-C)

| VIRUS | | | |
|---|---|---|---|
| | PK/15 | USA | France |
| PCV-T antiserum | ≥ 6 400 | 200 | 800 |
| PCV-C antiserum | 200 | ≥ 6,400 | ≥ 6,400 |
| F99 1H4 | ≥ 10 000 | < 100 | 100 |
| F99 4B10 | ≥ 10000 | < 100 | < 100 |
| F99 2B7 | ≥ 10 000 | 100 | < 100 |
| F99 2E12 | ≥ 10 000 | < 100 | < 100 |
| F99 1C9 | ≥ 10 000 | < 100 | 100 |
| F99 2E1 | ≥ 10 000 | < 100 | < 100 |
| F99 1H4 | ≥ 10 000 | 100 | < 100 |
| | | | |

| | | | |
|---|---|---|---|
| * inverse de la dernière dilution du sérum ou de l'anticorps monoclonal qui donne une réaction positive en immunofluorescence indirecte. | | | |

## Revendications

1. Vecteur d'expression comprenant une séquence nucléotidique d'un circovirus porcin de type II (PCV II) responsable du syndrome de dépérissement généralisé de post-sevrage (PMWS), dans lequel vecteur ladite séquence nucléotidique comprend un cadre ouvert de lecture (COL) du circovirus porcin de type II (PCV II) ledit cadre ouvert de lecture étant choisi dans le groupe constitué :
- du cadre ouvert de lecture 4 (COL 4) constitué des nucléotides 398 à 1342 du brin sens de la séquence référencée SEQ ID NO : 1, SEQ ID NO : 2, SEQ ID NO : 3, SEQ ID NO : 4 ou SEQ ID NO : 6,
- du cadre ouvert de lecture 13 (COL 13) constitué des nucléotides 314 à 1377 du brin antisens de la séquence référencée SEQ ID NO : 1 ou SEQ ID NO : 2 ou des nucléotides 314 à 1381 du brin antisens de la séquence référencée SEQ ID NO : 3, SEQ ID NO :4 ou SEQ ID NO : 6,
- du cadre ouvert de lecture 7 (COL 7) constitué des nucléotides 1018 à 704 du brin antisens de la séquence référencée SEQ ID NO : 1, SEQ ID NO : 2, SEQ ID NO : 3, SEQ ID NO : 4 ou SEQ ID NO : 6, et
- du cadre ouvert de lecture 10 (COL 10) constitué des nucléotides 912 à 733 du brin antisens de la séquence référencée SEQ ID NO : 1, SEQ ID NO : 2, SEQ ID NO : 3, SEQ ID NO : 4 ou SEQ ID NO : 6,
ledit vecteur d'expression exprimant *in vitro* ou *in vivo* ledit cadre ouvert de lecture du circovirus porcin de type II.

2. Vecteur d'expression selon la revendication 1, dans lequel vecteur la séquence nucléotidique comprend le cadre ouvert de lecture 4 (COL 4) ou le cadre ouvert de lecture 13 (COL 13).

3. Vecteur d'expression selon la revendication 1 ou la revendication 2, dans lequel le vecteur est un baculovirus ou un plasmide.

4. Vecteur d'expression selon la revendication 1 ou la revendication 2, dans lequel le vecteur est approprié pour l'expression *in vivo*.

5. Vecteur d'expression selon la revendication 4, dans lequel le vecteur est un virus vivant ou un plasmide.

6. Vecteur d'expression selon la revendication 5, dans lequel le vecteur est choisi parmi les herpès virus du porc, l'adénovirus porcin et les poxvirus.

7. Vecteur d'expression selon la revendication 6, dans lequel le vecteur est choisi parmi le virus de la maladie d'Aujeszky, le virus de la vaccine, l'avipox et le swinepox.

8. Vecteur d'expression selon la revendication 7, dans lequel le vecteur est le canarypox.

9. Vecteur d'expression selon l'une quelconque des revendications 4 à 8, dans lequel le vecteur d'expression est utilisé pour induire une réponse immunitaire chez le porc vis-à-vis d'un circovirus porcin de type II (PCV II).

10. Une cellule exprimant *in vitro* un polypeptide d'un circovirus porcin de type II (PCV II), la cellule comprenant un vecteur d'expression selon l'une quelconque des revendications 1 à 3.

11. Cellule selon la revendication 10, dans laquelle la cellule est une cellule *E*. *coli* ou une cellule eucaryote.

12. Cellule selon la revendication 11, dans laquelle la cellule eucaryote est une levure.

13. Cellule selon la revendication 11, dans laquelle la cellule eucaryote est une cellule de mammifère.

14. Cellule selon la revendication 11, dans laquelle la cellule eucaryote est une cellule d'insecte.

15. Polypeptide codé par un fragment d'ADN comprenant un cadre ouvert de lecture (COL) d'un circovirus porcin de type II (PCV II), le cadre ouvert de lecture étant choisi dans le groupe constitué :
- du cadre ouvert de lecture 4 (COL 4) constitué des nucléotides 398 à 1342 du brin sens de la séquence référencée SEQ ID NO : 1, SEQ ID NO : 2, SEQ ID NO : 3, SEQ ID NO : 4 ou SEQ ID NO : 6,
- du cadre ouvert de lecture 13 (COL 13) constitué des nucléotides 314 à 1377 du brin antisens de la séquence référencée SEQ ID NO : 1 ou SEQ ID NO : 2 ou des nucléotides 314 à 1381 du brin antisens de la séquence référencée SEQ ID NO : 3, SEQ ID NO : 4 ou SEQ ID NO : 6,
- du cadre ouvert de lecture 7 (COL 7) constitué des nucléotides 1018 à 704 du brin antisens de la séquence référencée SEQ ID NO : 1, SEQ ID NO : 2, SEQ ID NO : 3, SEQ ID NO: 4 ou SEQ ID NO : 6, et
- du cadre ouvert de lecture 10 (COL 10) constitué des nucléotides 912 à 733 du brin antisens de la séquence référencée SEQ ID NO : 1, SEQ ID NO : 2, SEQ ID NO : 3, SEQ ID NO : 4 ou SEQ ID NO : 6.

16. Polypeptide selon la revendication 15, dans lequel le cadre ouvert de lecture de PCV II est le cadre ouvert de lecture 4 (COL 4) ou le cadre ouvert de lecture 13 (COL13).

17. Polypeptide selon la revendication 15 ou la revendication 16, dans lequel le polypeptide est utilisé pour induire une réponse immunitaire chez le porc vis-à-vis d'un circovirus porcin de type II (PCV II).

18. Vaccin de sous-unité comprenant au moins un polypeptide la revendication 15 ou la revendication 16, dans un véhicule ou diluant acceptable sur le plan vétérinaire.

19. Vaccin de sous unité selon la revendication 18 comprenant en outre un adjuvant acceptable sur le plan vétérinaire.

20. Vaccin comprenant au moins un vecteur d'expression selon l'une quelconque des revendications 4 à 8 dans un véhicule ou diluant acceptable sur le plan vétérinaire.

## Patentansprüche

1. Expressionsvektor, umfassend eine Nukleotidsequenz eines porcinen Circovirus vom Typ II (PCV II), der für das verallgemeinerte Verfallssyndrom nach Entwöhnung (PMWS) verantwortlich ist, wobei in dem Vektor die genannte Nukleotidsequenz ein offenes Leseraster (OLR) des porcinen Circovirus vom Typ II (PCV II) umfasst, wobei das genannte offene Leseraster (OLR) aus der Gruppe ausgewählt ist, die aus
- dem offenen Leseraster 4 (OLR 4), das aus den Nukleotiden 398 bis 1342 des Sense-Strangs der Referenzsequenz SEQ ID NO:1, SEQ ID NO:2, SEQ ID NO:3, SEQ ID NO:4 oder SEQ ID NO:6 besteht,
- dem offenen Leseraster 13 (OLR 13), das aus den Nukleotiden 314 bis 1377 des Antisense-Strangs der Referenzsequenz SEQ ID NO:1 oder SEQ ID NO:2, oder aus den Nukleotiden 314 bis 1381 des Antisense-Strangs der Referenzsequenz SEQ ID NO:3, SEQ ID NO:4 oder SEQ ID NO:6 besteht,
- dem offenen Leseraster 7 (OLR 7), das aus den Nukleotiden 1018 bis 704 des Antisense-Strangs der Referenzsequenz SEQ ID NO:1, SEQ ID NO:2, SEQ ID NO:3, SEQ ID NO:4 oder SEQ ID NO:6 besteht, und
- dem offenen Leseraster 10 (OLR 10), das aus den Nukleotiden 912 bis 733 des Antisense-Strangs der Referenzsequenz SEQ ID NO:1, SEQ ID NO:2, SEQ ID NO:3, SEQ ID NO:4 oder SEQ ID NO:6 besteht,
besteht, wobei der genannte Expressionsvektor das genannte offene Leseraster des porcinen Circovirus vom Typ II in vitro oder in vivo exprimiert.

2. Expressionsvektor nach Anspruch 1, wobei in dem Vektor die Nukleotidsequenz das offene Leseraster 4 (OLR 4) oder das offene Leseraster 13 (OLR 13) umfasst.

3. Expressionsvektor nach Anspruch 1 oder nach Anspruch 2, wobei der Vektor ein Baculovirus oder ein Plasmid ist.

4. Expressionsvektor nach Anspruch 1 oder nach Anspruch 2, wobei der Vektor für die Expression in vivo geeignet ist.

5. Expressionsvektor nach Anspruch 4, wobei der Vektor ein lebender Virus oder ein Plasmid ist.

6. Expressionsvektor nach Anspruch 5, wobei der Vektor aus Schweineherpesviren, Schweine-Adenoviren und Pockenviren ausgewählt ist.

7. Expressionsvektor nach Anspruch 6, wobei der Vektor aus dem Virus der Aujeszky-Krankheit, Vacciniavirus, Avipoxvirus und Schweinepockenvirus ausgewählt ist.

8. Expressionsvektor nach Anspruch 7, wobei der Vektor Canarypox ist.

9. Expressionsvektor nach einem der Ansprüche 4 bis 8, wobei der Expressionsvektor zum Hervorrufen einer Immunantwort in einem Schwein gegenüber einem porcinen Circovirus vom Typ II (PCV II) verwendet wird.

10. Zelle, die in vitro ein Polypeptid eines porcinen Circovirus vom Typ II (PCV II) exprimiert, wobei die Zelle einen Expressionsvektor nach einem der Ansprüche 1 bis 3 umfasst.

11. Zelle nach Anspruch 10, wobei die Zelle eine E. coli-Zelle oder eine eukaryotische Zelle ist.

12. Zelle nach Anspruch 11, wobei die eukaryotische Zelle eine Hefe ist.

13. Zelle nach Anspruch 11, wobei die eukaryotische Zelle eine Säugerzelle ist.

14. Zelle nach Anspruch 11, wobei die eukaryotische Zelle eine Insektenzelle ist.

15. Polypeptid, kodiert durch ein DNA-Fragment, umfassend ein offenes Leseraster (OLR) eines porcinen Circovirus vom Typ II (PCV II), wobei das offene Leseraster (OLR) aus der Gruppe ausgewählt ist, die aus
- dem offenen Leseraster 4 (OLR 4), das aus den Nukleotiden 398 bis 1342 des Sense-Strangs der Referenzsequenz SEQ ID NO:1, SEQ ID NO:2, SEQ ID NO:3, SEQ ID NO:4 oder SEQ ID NO:6 besteht,
- dem offenen Leseraster 13 (OLR 13), das aus den Nukleotiden 314 bis 1377 des Antisense-Strangs der Referenzsequenz SEQ ID NO:1 oder SEQ ID NO:2, oder aus den Nukleotiden 314 bis 1381 des Antisense-Strangs der Referenzsequenz SEQ ID NO:3, SEQ ID NO:4 oder SEQ ID NO:6 besteht,
- dem offenen Leseraster 7 (OLR 7), das aus den Nukleotiden 1018 bis 704 des Antisense-Strangs der Referenzsequenz SEQ ID NO:1, SEQ ID NO:2, SEQ ID NO:3, SEQ ID NO:4 oder SEQ ID NO:6 besteht, und
- dem offenen Leseraster 10 (OLR 10), das aus den Nukleotiden 912 bis 733 des Antisense-Strangs der Referenzsequenz SEQ ID NO:1, SEQ ID NO:2, SEQ ID NO:3, SEQ ID NO:4 oder SEQ ID NO:6 besteht,
besteht.

16. Polypeptid nach Anspruch 15, wobei das offene Leseraster von PCV II das offene Leseraster 4 (OLR 4) oder das offene Leseraster 13 (OLR 13) ist.

17. Polypeptid nach Anspruch 15 oder nach Anspruch 16, wobei das Polypeptid zum Hervorrufen einer Immunantwort in einem Schwein gegenüber einem porcinen Circovirus vom Typ II (PCV II) verwendet wird.

18. Untereinheit-Impfstoff, umfassend wenigstens ein Polypeptid nach Anspruch 15 oder nach Anspruch 16 in einem veterinärmedizinisch annehmbaren Vehikel oder Verdünnungsmittel.

19. Untereinheit-Impfstoff nach Anspruch 18, umfassend unter anderem ein veterinärmedizinisch annehmbares Adjuvans.

20. Impfstoff, umfassend wenigstens einen Expressionsvektor nach einem der Ansprüche 4 bis 8 in einem veterinärmedizinisch annehmbaren Vehikel oder Verdünnungsmittel.

## Claims

1. Expression vector comprising a nucleotidic sequence of a type II porcine circovirus (PCV II) responsible for post-weaning multisystemic wasting syndrome (PMWS), wherein said vector said nucleotidic sequence comprises an open reading frame (ORF) of the type II porcine circovirus (PCV II) said open reading frame being selected from the group consisting of:
- the open reading frame 4 (ORF 4) comprised of nucleotides 398 to 1342 of the sense strand of the sequence entered under the reference SEQ ID NO:1, SEQ ID NO:2, SEQ ID NO:3, SEQ ID NO:4 or SEQ ID NO:6,
- the open reading frame 13 (ORF 13) comprised of nucleotides 314 to 1377 of the antisense strand of the sequence entered under the reference SEQ ID NO: 1 or SEQ ID NO:2 or of nucleotides 314 to 1381 of the antisense strand of the sequence entered under the reference SEQ ID NO:3, SEQ ID NO:4 or SEQ ID NO:6,
- the open reading frame 7 (ORF 7) comprised of nucleotides 1018 to 704 of the antisense strand of the sequence entered under the reference SEQ ID NO:1, SEQ ID NO:2, SEQ ID NO:3, SEQ ID NO:4 or SEQ ID NO:6, and
- the open reading frame 10 (ORF 10) comprised of nucleotides 912 to 733 of the antisense strand of the sequence entered under the reference SEQ ID NO:1, SEQ ID NO:2, SEQ ID NO:3, SEQ ID NO:4 or SEQ ID NO:6,
said expression vector expressing in vitro or in vivo said open reading frame of the type II porcine circovirus.

2. Expression vector according to claim 1, wherein said vector the nucleotidic sequence comprises the open reading frame 4 (ORF 4) or the open reading frame 13 (ORF 13).

3. Expression vector according to claim 1 or claim 2, wherein the vector is a baculovirus or a plasmid.

4. Expression vector according to claim 1 or claim 2, wherein the vector is suitable for expressing in vivo.

5. Expression vector according to claim 4, wherein the vector is a live virus or a plasmid.

6. Expression vector according to claim 5, wherein the vector is selected from among porcine herpes viruses, porcine adenovirus and poxviruses.

7. Expression vector according to claim 6, wherein the vector is selected from among Aujeszky's disease virus, vaccinia virus, avipox virus and swinepox virus.

8. Expression vector according to claim 7, wherein the vector is the canarypox virus.

9. Expression vector according to any of claims 4 to 8, wherein the expression vector is used to induce an immune response in pigs against a type II porcine circovirus (PCV II).

10. A cell expressing in vitro a polypeptide of a type II porcine circovirus (PCV II), the cell comprising an expression vector according to any of claims 1 to 3.

11. Cell according to claim 10, wherein the cell is an E. coli cell or a eukaryotic cell.

12. Cell according to claim 11, wherein the eukaryotic cell is a yeast.

13. Cell according to claim 11, wherein the eukaryotic cell is a mammalian cell.

14. Cell according to claim 11, wherein the eukaryotic cell is an insect cell.

15. Polypeptide encoded by a DNA fragment comprising an open reading frame (ORF) of a type II porcine circovirus (PCV II), the open reading frame being selected from the group consisting of:
- the open reading frame 4 (ORF 4) comprised of nucleotides 398 to 1342 of the sense strand of the sequence entered under the reference SEQ ID NO:1, SEQ ID NO:2, SEQ ID NO:3, SEQ ID NO:4 or SEQ ID NO:6,
- the open reading frame 13 (ORF 13) comprised of nucleotides 314 to 1377 of the antisense strand of the sequence entered under the reference SEQ ID NO:1 or SEQ ID NO:2 or of nucleotides 314 to 1381 of the antisense strand of the sequence entered under the reference SEQ ID NO:3, SEQ ID NO:4 or SEQ ID NO:6,
- the open reading frame 7 (ORF 7) comprised of nucleotides 1018 to 704 of the antisense strand of the sequence entered under the reference SEQ ID NO:1, SEQ ID NO:2, SEQ ID NO:3, SEQ ID NO:4 or SEQ ID NO:6, and
- the open reading frame 10 (ORF 10) comprised of nucleotides 912 to 733 of the antisense strand of the sequence entered under the reference SEQ ID NO:1, SEQ ID NO:2, SEQ ID NO:3, SEQ ID NO:4 or SEQ ID NO:6.

16. Polypeptide according to claim 15, wherein the open reading frame of PCV II is the open reading frame 4 (ORF 4) or the open reading frame 13 (ORF 13).

17. Polypeptide according to claim 15 or claim 16, wherein the polypeptide is used to induce an immune response in pigs against a type II porcine circovirus (PCV II).

18. Subunit vaccine comprising at least one polypeptide according to claim 15 or claim 16, in veterinarily acceptable vehicle or diluent.

19. Subunit vaccine according to claim 18 further comprising a veterinarily acceptable adjuvant.

20. Vaccine comprising at least one expression vector according to any of claims 4 to 8 in a veterinarily acceptable vehicle or diluent.
